# EUROPÄISCHE PATENTSCHRIFT

(11) **EP 1 036 540 B1**
(45) Veröffentlichungstag und Bekanntmachung des Hinweises auf die Patenterteilung: **16.03.2005**
(21) Anmeldenummer: 00890082.1
(22) Anmeldetag: 16.03.2000
(51) Int. Cl.: A61B 5/0416

(54) **Medizinische Elektrode**
Medical electrode
Electrode médicale

(30) Priorität: 16.03.1999 AT 17599 U
(43) Veröffentlichungstag der Anmeldung: 20.09.2000
(73) Patentinhaber: Nessler Medizintechnik GmbH & Co KG, 6020 Innsbruck (AT)
(72) Erfinder: Nessler, Winfried Dr., 81739 München (DE)
(74) Vertreter: Ellmeyer, Wolfgang

(56) Entgegenhaltungen:
- DE-A- 4 410 017
- US-A- 3 805 769

## Beschreibung

Die vorliegende Erfindung betrifft medizinische Elektroden mit einer rauhen Stimulierzone zur Förderung der Durchblutung und zum Reinigen und Aufrauhen der Haut an der Kontaktstelle.

Insbesondere bei EKG-Elektroden ist es teilweise üblich, Mittel zur Durchblutungsförderung, Reinigung und Aufrauhung der Haut (aus dem Englischen entlehnt als "Abrader" bezeichnet) an der Kontaktstelle beizupacken bzw. an einem Teil der Elektrode vorzusehen. Beispielsweise existieren von der Minnesota Mining & Manufacturing Company (3M) Elektroden (Handelsname Red Dot™), bei denen ein Stück Schleifpapier an die Unterseite aufgeklebt ist. Dieses stellt eine zusätzliche Materialkomponente dar und erhöht den Aufwand und die Kosten des Herstellungsverfahrens und der Elektrode selbst.

Derartige Elektroden werden beispielsweise auch in der US-A-3.805.769 offenbart, wo anstelle des Schleifpapiers ein "Reinigungsstreifen", z.B. aus Gummi, an der Unterseite der Elektrode angebracht ist.

Unter dem Handelsnamen Profile™ sind Elektroden bekannt, wobei ein die Elektrodenfläche überragender, im wesentlichen rechteckiger, ebener Kunststoff-Liner in der Nähe des Randes mit einer aufgerauhten Stimulierzone versehen ist. Eine ähnliche Konstruktion ist von Nikomed erhältlich, wobei der (hier kreisrunde) Liner allerdings einen zentralen erhabenen Schutzbereich für die Elektrodenzone aufweist. Die Stimulierzone ist jedoch auch hier (in diesem Fall sogar direkt) am Rand des Liners vorgesehen.

Bei Elektroden dieses Typs können zwar zusätzliche Komponenten, wie etwa bei der 3M-Elektrode, entfallen, allerdings wird die Stimulierzone durch Perforieren bzw. Prägen nach der eigentlichen Herstellung des Liners, also in einem gesonderten Arbeitsgang, vorgesehen, was wiederum die Herstellung aufwendiger und teurer macht und eine zusätzliche mögliche Fehlerquelle bei der Herstellung schafft (z.B. können die Perforationsnadeln steckenbleiben, oder der Liner kann, falls die Perforation erst nach der Assemblierung der gesamten Elektrode erfolgt, von dieser abreißen). Weiters können die Kanten der harten Abdeckfolie beim Reiben an der Haut des Patienten diese verletzen, oder, falls die Behandlung der Haut vor dem Abziehen des Liners erfolgt (was der Normalfall ist), kann die Elektrodenzone durch Biegen oder Knicken in Mitleidenschaft gezogen werden, sodaß keine zuverlässige Leistung der Elektrode gewährleistet ist.

Die DE 44 10 017 A1 offenbart schließlich eine profilierte Biosignal-Elektrodenvorrichtung mit einem gegenüber dem Rest des Substrats erhabenen Sensor, der zusätzlich mit sogenannten "Riffelungen" oder durchbrochenen Punkten versehen ist, um aufgrund teilweiser Durchdringung der Epidermis mit den feinen Punkten ein Verrutschen der Elektrode zu verhindern. Eine Reinigung oder ein Aufrauhen der Haut mit diesen Riffelungen wird nicht erwähnt.

Die vorliegende Erfindung trachtet danach, die Probleme nach dem Stand der Technik zu lösen, indem eine Elektrode bereitgestellt werden soll, in der die Stimulierzone einstückig in einem Teil der Elektrode so vorgesehen ist, dass eine für den Patienten gefahrlose Verwendung derselben gewährleistet wird, ohne die Elektrode zu beschädigen, und die auf kostengünstige Weise herstellbar ist.

Dies erfolgt erfindungsgemäß durch Bereitstellung einer medizinischen Elektrode, insbesondere einer gelhältigen EKG-Elektrode, die einen flächigen, elektrisch nichtleitenden Träger mit einer Haftschicht zum Aufkleben auf die Hautoberfläche, eine kreisförmige Elektrodenzone zum Hautkontakt, vorzugsweise ein mit elektrisch leitendem Gel imprägniertes Schwämmchen, einen damit verbundenen Kontaktknopf zum Anschließen eines Kabels sowie eine vor Gebrauch abzuziehende isolierende Schutzabdeckung für die Elektrodenzone umfaßt, wobei die Schutzabdeckung mit einer rauhen Stimulierzone zum Aufrauhen und zur Durchblutungsförderung der Haut versehen ist, wobei die erfindungsgemäße Elektrode dadurch gekennzeichnet ist, daß die Stimulierzone in einem gegenüber dem Rest der Schutzabdeckung erhabenen, direkt mit der Elektrodenzone in Kontakt stehenden Teil der Schutzabdeckung vorgesehen ist. In einer bevorzugten Ausführungsform ist bzw. sind der Rand bzw. die Ränder der Stimulierzone zusätzlich abgerundet.

Durch eine derartige Elektroden-Konstruktion wird gewährleistet, daß die Haut des Patienten beim Anreiben mit der in der Schutzabdeckung enthaltenen Stimulierzone mit keinerlei scharfen Kanten oder Rändern in Kontakt kommt und daß damit Druck auf die Haut ausgeübt werden kann, ohne die Elektrode zu verbiegen, wodurch a) Verletzungen des Patienten (z.B. Schnitte), b) Schäden an der Elektrode und c) der Einsatz zusätzlicher Elektroden-Komponenten wirksam vermieden werden können.

In einer weiteren bevorzugten Ausführungsform ist die erfindungsgemäße Elektrode dadurch gekennzeichnet, daß der erhabene Teil eine zentrale Vertiefung zur Beherbergung eines etwaigen herstellungsbedingten Angusses bzw. Grates aufweist, wobei die Stimulierzone um die Vertiefung herum angeordnet ist. Auf diese Weise wird sichergestellt, daß auch ein derartiger Anguß oder Grat beim Anreiben der Haut keine Verletzungen hervorruft, ohne aufwendig entfernt werden zu müssen.

Die Schutzabdeckung inklusive der Stimulierzone der erfindungsgemäßen Elektrode ist vorzugsweise durch Spritzguß bzw. Tiefziehen in einem Arbeitsgang hergestellt, wodurch der Aufwand und die Kosten bei der Herstellung gegenüber dem Stand der Technik deutlich gesenkt werden können.

In einem zweiten Aspekt bietet die Erfindung ein Herstellungsverfahren für letztere bevorzugte Ausführungsform der Schutzabdeckung der erfindungsgemäßen Elektrode durch Spritzguß bzw. Tiefziehen, welches sich dadurch auszeichnet, daß die Form bzw. der Prägekopf einen gerauhten Abschnitt aufweist, um in einem Arbeitsgang die Stimulierzone in der Schutzabdeckung vorzusehen, sowie daß gleichzeitig damit eine zentrale Vertiefung zur Beherbergung eines Angusses bzw. Grates in die Schutzabdeckung gegossen bzw. geprägt wird, was -- wie oben erwähnt -- die Herstellungskosten der erfindungsgemäßen Elektrode beträchtlich verringert.

Nachstehend wird eine bevorzugte Ausführungsform der Erfindung anhand der beigelegten Zeichnungen näher beschrieben, wovon
Fig. 1 eine schematische Explosions-Querschnittsansicht einer herkömmlichen EKG-Elektrode ist; und
Fig. 2 eine vergrößerte schematische Querschnittsansicht einer Schutzabdeckung mit Stimulierzone gemäß vorliegender Erfindung ist.

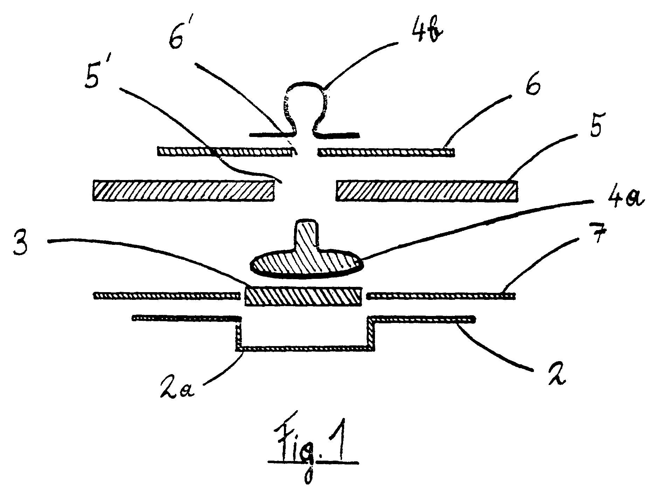

Fig. 1 zeigt schematisch den Aufbau einer EKG-Elektrode nach dem Stand der Technik in Form einer Explosionsansicht. Den Kern der Elektrode bildet eine, hier bevorzugt zentral angeordnete, Elektrodenzone 3, die vorzugsweise aus einem mit Elektrolyt, insbesondere elektrisch leitfähigem Gel, getränkten Schwämmchen besteht. Diese steht in leitendem Kontakt mit einem ebenfalls zentrierten Kontaktknopf 4a, 4b, in Form einer aus einem Nietstift 4a und einem Anschlußkopf 4b bestehenden Nietverbindung.

Der Anschlußkopf 4b wird vorzugsweise aus widerstandsfähigem Edelstahl gefertigt und dient zur Befestigung der (nicht dargestellten) Anschlußkabel an der Elektrode.

Der Nietstift 4a besteht üblicherweise aus Silber oder vorzugsweise aus Kunststoff, z.B. ABS-Polymer, mit galvanischem Silber-Überzug. Zumindest an der Unterseite ist (in Fig. 1 durch die dickere Strichführung angedeutet) eine Silberchloridschicht (durch Chlorieren des Silber-Überzugs) vorgesehen, um Schwankungen der elektrischen Leitung von Elektrodenzone 3 zum Anschlußkopf 4b auszugleichen. In der Praxis kann eine Silberchlondschicht an der gesamten Oberfläche ausgebildet sein.

Durch diesen Kontaktknopf 4a, 4b miteinander vernietet sind ein Träger 5 aus einseitig (an der Unterseite) klebendem Schaumstoff, der üblicherweise aus einer Endlosbahn ausgestanzt wird und vorzugsweise kreisrunde oder mehr bevorzugt quadratische Form mit abgerundeten Ecken aufweist, und ein Etikett 6, dessen Form an jene des Trägers angepaßt ist und das zusätzlich zur Nietverbindung noch mit dem Träger 5 verklebt ist. Das Etikett 6 kann den Handelsnamen des Produkts, den Namen des Herstellers, Angaben zur Elektrode oder dergleichen tragen und besteht im allgemeinen aus einseitig klebender Kunststoff-Folie.

Die Elektrodenzone 3 ist -- wie aus Fig. 1 zu entnehmen -- unterhalb des Nietstifts 4a angeordnet und in der Praxis auf das Etikett 6 aufgeklebt. Gegenüber Fig. 1 sind dann die Öffnung 5' im Träger 5 und die Elektrodenzone 3 entsprechend größer, sodaß die Elektrodenzone 3 in der Öffnung 5' sitzt und neben dem Nietstift 4a am Etikett 6 klebt.

Der Nietstift 4a ist durch Öffnungen 5' bzw. 6' von Träger 5 und Etikett 6 hindurchgeführt, um oberhalb des Etiketts 6 in den Anschlußkopf 4b einzugreifen, wodurch die Nietverbindung hergestellt wird. In Fig. 1 ist weiters eine (eingangs auch als Liner bezeichnete) dünne, die Elektrodenzone 3 umgebende, ablösbare Abziehfolie 7 zum Schutz der klebenden Unterseite des Trägers 5, sowie eine zusammen mit der Folie 7 ablösbare, die Elektrodenzone 3 abschirmende Schutzabdeckung 2 dargestellt, die um die Elektrodenzone 3 herum auf die Abziehfolie 7 aufgeklebt ist.

Diese Schutzabdeckung 2 umfaßt einen erhabenen Teil 2a, der in direktem Kontakt mit der Elektrodenzone 3 steht, um deren Schutz beim Manipulieren der Elektrode zu gewährleisten. Obwohl in Fig. 1 eckig dargestellt, kann die Schutzabdeckung nach dem Stand der Technik auch abgerundete Kanten aufweisen.

Die eingangs beschriebenen Stimulierzonen der Marken Profile™ und Nikomed befinden sich in einem Fall direkt an der Abziehfolie (Liner) 7, da die Profile™-Elektrode gar keine gesonderte Schutzabdeckung 2 vorsieht sondern ein ebener Liner 7 die gesamte Unterseite der Elektrode (also auch die Elektrodenzone 3) abdeckt, bzw. im Fall der Nikomed-Elektrode am flachen, direkt am Liner 7 anliegenden und -haftenden Teil der Schutzabdeckung 2. Wie bereits erwähnt kommt in beiden Fällen die Haut des Patienten beim Anreiben derselben fast zwangsläufig mit dem Rand des Liners 7 bzw. der Schutzabdeckung 2 in Kontakt, was potentielle Verletzungsgefahren (z.B. durch Schnitte) mit sich bringt, und falls das Anreiben der Haut vor dem Abziehen des Liners 7 bzw. der Schutzabdeckung 2 erfolgt, wird die Elektrode vom Anwender (Arzt, Krankenschwester) meist verbogen und kann Schäden davontragen.

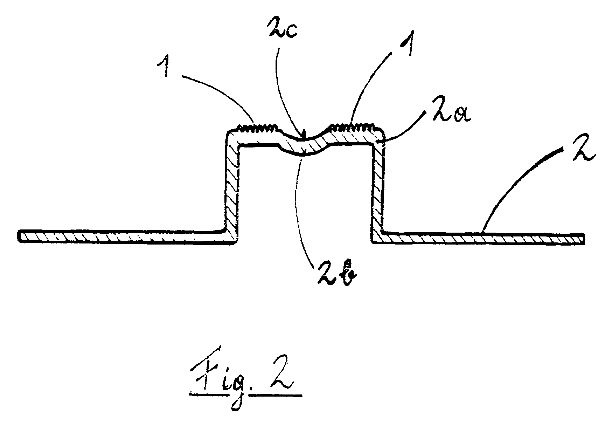

Die erfindungsgemäße Lösung dieser Probleme ist nun in Fig. 2 dargestellt, die eine besonders bevorzugte Ausführungsform der Schutzabdeckung 2 der Erfindung zeigt. Eine solche Schutzabdeckung 2 umfaßt eine rauhe Stimulierzone 1 an der Deckfläche ihres erhabenen Teils 2a; diese befindet sich vor dem Abziehen also unmittelbar unterhalb der Elektrodenzone 3. Dadurch besteht beim Anreiben der Haut des Patienten -- sowohl vor als auch nach dem Abziehen von Schutzabdeckung 2 und Abziehfolie/Liner 7 - kaum Verletzungsgefahr für den Patienten noch die Gefahr von Beschädigung der Elektrode selbst, und die Ausübung von Druck auf die Haut wird deutlich erleichtert.

Erfindungsgemäß bevorzugt ist der Rand (im Falle von runden oder kreisförmigen Schutzabdeckungen) bzw. sind die Ränder (bei eckigen, z.B. quadratischen, Ausführungsformen) des erhabenen Teils 2a, d.h. der Deckfläche, in Fig. 2 zusätzlich abgerundet, und es ist als besonders bevorzugtes Merkmal eine Vertiefung 2b in der Deckfläche vorgesehen, in der sich ein beim Spritzgießen üblicherweise anfallender Anguß oder Grat 2c befindet, sodaß der Grat 2c beim Anreiben der Haut mit der Stimulierzone 1 nicht in Kontakt mit der Haut kommt, wodurch Verletzungsgefahren gänzlich ausgeschaltet werden, ohne den Grat 2c in einem gesonderten Arbeitsschritt entfernen zu müssen. Die Stimulierzone 1 ist somit um die Vertiefung 2b herum, bei kreisförmigen Schutzabdeckungen z.B. in Form eines Kreisrings, angeordnet.

Zur Kostenminimierung wird die erfindungsgemäße Schutzabdeckung 2 mit Stimulierzone 1 in einem Arbeitsgang entweder durch Spritzguß in einer der Stimulierzone 1 entsprechend teilweise aufgerauhten Form oder durch Tiefziehen mit einem entsprechend rauhen Prägekopf hergestellt, was ebenfalls eine deutliche Verbesserung gegenüber dem Stand der Technik (Beilegen einer zusätzlichen Komponente bzw. nachträgliches Prägen/Perforieren von Liner 7 oder Schutzabdeckung 2) darstellt.

## Patentansprüche

1. Medizinische Elektrode, insbesondere gelhältige EKG-Elektrode, umfassend einen flächigen, elektrisch nichtleitenden Träger mit einer Haftschicht zum Aufkleben auf die Hautoberfläche, eine kreisförmige Elektrodenzone zum Hautkontakt, vorzugsweise ein mit elektrisch leitendem Gel imprägniertes Schwämmchen, einen damit verbundenen Kontaktknopf zum Anschließen eines Kabels sowie eine vor Gebrauch abzuziehende isolierende Schutzabdeckung für die Elektrodenzone, wobei die Schutzabdeckung mit einer rauhen Stimulierzone zum Aufrauhen und zur Durchblutungsförderung der Haut versehen ist,
**dadurch gekennzeichnet, daß** die Stimulierzone (1) in einem gegenüber dem Rest der Schutzabdeckung (2) erhabenen, direkt mit der Elektrodenzone (3) in Kontakt stehenden Teil (2a) der Schutzabdeckung (2) vorgesehen ist.

2. Elektrode nach Anspruch 1, **dadurch gekennzeichnet, daß** der Rand bzw. die Ränder der Stimulierzone (1) abgerundet ist bzw. sind.

3. Elektrode nach Anspruch 1 oder 2, **dadurch gekennzeichnet, daß** der erhabene Teil (2a) eine zentrale Vertiefung (2b) zur Beherbergung eines etwaigen herstellungsbedingten Angusses bzw. Grates (2c) aufweist, wobei die Stimulierzone (1 ) um die Vertiefung (2b) herum angeordnet ist.

4. Elektrode nach einem der Ansprüche 1 bis 3, **dadurch gekennzeichnet, daß** die Schutzabdeckung (2) inklusive der Stimulierzone (1) durch Spritzguß bzw. Tiefziehen in einem Arbeitsgang hergestellt ist.

5. Verfahren zur Herstellung einer Elektrode nach Anspruch 3, umfassend die Herstellung der Schutzabdeckung (2) durch Spritzguß bzw. Tiefziehen, **dadurch gekennzeichnet, daß** die Form bzw. der Prägekopf einen gerauhten Abschnitt aufweist, um in einem Arbeitsgang die Stimulierzone (1) in der Schutzabdeckung (2) vorzusehen, sowie daß gleichzeitig damit eine zentrale Vertiefung (2b) zur Beherbergung eines Angusses bzw. Grates (2c) in die Schutzabdeckung (2) gegossen bzw. geprägt wird.

## Claims

1. A medical electrode, particularly a gel-containing ECG electrode, comprising: a flat, non-electroconducting carrier having an adhesive surface for adhering the electrode onto the skin surface; a circular electrode zone for contacting the skin, preferably a small sponge impregnated with an electroconducting gel; a contact button for connecting a cable; and an isolating protection cover to be detached before use, the protection cover being provided with a rough stimulation zone for roughening the skin and stimulating blood circulation in the skin,
**characterized in that** the stimulation zone (1) is provided in a part (2a) of the protection cover (2), which is raised compared to the remainder of the protection cover (2) and is in direct contact with the electrode zone (3).

2. The electrode according to claim 1, **characterized in that** the edge(s) of the stimulation zone (1) is/are rounded.

3. The electrode according to claim 1 or 2, **characterized in that** the raised part (2a) has a central depression (2b) for receiving any fabrication-related burr or fin (2c), the stimulation zone (1) being located around the depression (2b).

4. The electrode according to any of claims 1 to 3, **characterized in that** the protection cover (2), including the stimulation zone (1), is produced by single-operation injection moulding or deep drawing.

5. A method for producing an electrode according to claim 3, comprising the production of the protection cover (2) by injection moulding or deep drawing, **characterized in that** the mould or the punch, respectively, has a roughened portion for providing the stimulation zone (1) in the protection cover (2) in a single operation and **in that**, simultaneously, a central depression (2b) for receiving a burr or fin (2c) is cast or embossed into the protection cover (2).

## Revendications

1. Electrode médicale, en particulier électrode d'ECG contenant du gel, comprenant: un support isolant plat ayant une couche adhérente afin de la coller à la surface de la peau; une zone circulaire d'électrode pour le contact cutané, de préférence un petit éponge imprégné d'un gel conducteur; un bouton de contact lié avec ce dernier pour établir une connexion avec un cable; ainsi qu'un revêtement de protection isolant détachable pour la zone d'électrode, le revêtement étant pourvu d'une zone de stimulation râpeuse pour rendre la peau rugueuse et stimuler la circulation sanguine de la peau,
**caractérisée en ce que** la zone de stimulation (1) est prévue dans une partie (2a) du revêtement de protection (2), qui fait saillie comparée au reste du revêtement de protection (2) et qui est en contact direct avec la zone d'électrode (3).

2. Electrode selon la revendication 1, **caractérisée en ce que** le bord ou les bords de la zone de stimulation (1) est/sont arrondi(s).

3. Electrode selon la revendication 1 ou 2, **caractérisée en ce que** la partie qui fait saillie (2a) représente une concavité centrale (2b) pour accueillir une bavure ou une arête (2c), formée éventuellement lors de la fabrication, la zone de stimulation (1) se trouvant autour de cette concavité (2b).

4. Electrode selon l'une quelconque des revendications 1 à 3, **caractérisée en ce que** le revêtement de protection (2) et la zone de stimulation (1) sont fabriqués par des procédés de moulage par injection ou d'emboutissage profond en une phase de fabrication.

5. Procédé de fabrication d'une électrode selon la revendication 3, comprenant la fabrication du revêtement de protection (2) par moulage par injection ou emboutissage profond, **caractérisé en ce que** le moule ou l'emboutisseur représente une partie rugueuse afin de former la zone de stimulation (1) dans le revêtement de protection (2) lors d'une seule phase de fabrication, ainsi que, concurremment, une concavité centrale (2b) est moulée ou emboutie dans le revêtement de protection (2) afin d'accueillir une bavure ou une arête (2c).
